# EUROPEAN PATENT APPLICATION

(11) **EP 2 005 952 A1**
(43) Date of publication of application: **24.12.2008**
(21) Application number: 07740885.4
(22) Date of filing: 03.04.2007
(51) Int. Cl.: A61K 31/18, A61K 9/70, A61K 47/10, A61K 47/14, A61K 47/32, A61P 7/10, A61P 13/08, A61P 43/00

(54) **TAMSULOSIN-CONTAINING PERCUTANEOUS ABSORPTION TYPE PREPARATION**

(30) Priority: 11.04.2006 JP 2006108915
(71) Applicant: Nichiban Company Limited, Bunkyo-ku, Tokyo 112-8663 (JP)
(72) Inventor: KAWAHARA, Kouji, Tokyo 112-8663 (JP); NAKAO, Kenichi, Tokyo 112-8663 (JP); YAMAKI, Haruna, Tokyo 112-8663 (JP)
(74) Representative: Senior, Janet
(86) International application number: PCT/JP2007/057449
(87) International publication number: WO 2007/119656

(57) **Abstract**

A tamsulosin-containing percutaneous absorption type preparation. A percutaneous absorption type preparation comprising a support and an adhesive layer on the support, **characterized in that** the adhesive layer contains an acrylic adhesive, further contains tamsulosin as an active ingredient in an amount of 0.1 to 20% by mass of the total mass of the adhesive layer and, if desired, a polyoxyethylene alkyl ether which is an additive playing a role in increasing solubility and a propylene glycol fatty acid ester which is an additive playing a role in promoting percutaneous absorption is provided. Further, a percutaneous absorption type preparation comprising a support and an adhesive layer on the support **characterized in that** the adhesive layer contains a synthetic rubber adhesive, further contains tamsulosin as an active ingredient in an amount of 0.1 to 20% by mass of the total mass of the adhesive layer and a propylene glycol fatty acid ester which is an additive playing a role in promoting percutaneous absorption is provided.

## Description

### TECHNICAL FIELD

The present invention relates to a transdermal patch for causing a patient to absorb percutaneously tamsulosin ((-)-(R)-5-(2-((2-(o-ethoxyphenoxy)ethyl)amino)propyl)-2-methoxybenzenesulfonamidehydrochloride) which is a drug for improving dysuria caused by benign prostatic hyperplasia.

### BACKGROUND ART

Benign prostatic hyperplasia is a disease in which the dysuria is caused by a physical pressure of the urethra caused due to the hypertrophy of the prostate which is an organ under the bladder and by a functional contraction of the prostate and the urethra caused due to the sympathetic hyperactivity. It is said that at present, one fifth of men at the age of 55 and older is a patient thereof when including potential patients.

Tamsulosin is an α1-adrenoceptor antagonist having a strong relaxant effect of a smooth muscle of a lower urinary tract. When the drug is administered in the human body, it loosens the urethra, increases the flow of urine and improves the symptom of constant urge to urinate or pollakiuria, so that it is effective for treating the dysuria caused by benign prostatic hyperplasia.
Conventionally, as the method for administrating tamsulosin, an oral administration is adopted, however, in recent years, a method for administrating tamsulosin using a transdermal patch is investigated.
The administration method using a transdermal patch can solve a problem of the oral administration, that is, can prevent high doses due to the metabolism (first-pass effect) of a drug in the alimentary tract or liver, and moreover, by this administration method, the drug effect continues for a long period, which leads to a reduction of the number of doses. In addition, the administration can be so simply performed as only by "applying", so that there can also be mentioned as advantages thereof, improvement of the compliance, and easiness of initiating and discontinuing the administration. Further, though in the case of the oral administration, the change in the blood level of the drug due to the influence of the meal should be taken into consideration, the percutaneous administration is not subject to such an influence of the meal, so that it has such an advantage that it can be performed in peace.
Under such circumstances, particularly for a patient of benign prostatic hyperplasia in which the higher the patient is aged, the more the incidence rate is, the transdermal patch is expected as a useful drug administration method uniting convenience and effectiveness.

Generally, the transdermal patch such as an adhesive tape preparation is that in which an adhesive layer containing a drug is disposed on a backing-layer. In causing a patient to absorb percutaneously tamsulosin using a transdermal patch, what is first to be taken into consideration is that since a configuration of tamsulosin-base (tamsulosin free form) is crystalline, when tamsulosin is used as it is, it becomes to exist in a crystalline state in the adhesive layer, so that it is necessary to dissolve it at least partially before the use thereof. Further, tamsulosin is slightly-soluble in many solvents, so that it becomes important how to increase the solubility thereof.
Various methods for producing a transdermal patch by improving such a slight-solubility of tamsulosin in various solvents, particularly the solubility thereof relative to an adhesive, are published.
For example, disclosed are a method for producing a transdermal patch incorporating tamsulosin in an adhesive containing as main bases, a low molecular weight-polyisobutylene, a high molecular weight-polyisobutylene and an oil (Patent Document 1) and a method for producing a percutaneous absorption formulation containing tamsulosin-base and capable of using any one of acrylic, rubber and silicone adhesives (Patent Documents 2 and 3). Particularly, in Patent Documents 2 and 3, disclosed are a percutaneous absorption type formulation in which the acrylic adhesive such as polyaminoacrylate aqueous solution containing a fatty acid and higher fatty acid esters as an agent for promoting percutaneous absorption of tamsulosin are combined, and a percutaneous absorption preparation in which an emulsion-type acrylic adhesive emulsion-polymerized in an aqueous solution and ethanol or glycols as a solubilizer of tamsulosin are combined.
Patent Document 1: WO 95/31190 pamphlet
Patent Document 2: Japanese Patent Application Publication No. JP-A-8-92080
Patent Document 3: Japanese Patent Application Publication No. JP-A-8-245377

### DISCLOSURE OF THE INVENTION

### [Problems to be Solved by the Invention]

However, in preparations using a rubber adhesive or a silicone adhesive which have been proposed hitherto, the skin permeability of tamsulosin is not satisfactory. Even in an emulsion-type acrylic adhesive in which the skin permeability has been improved from that in the preparations, there is room for further improving the skin permeability.
In addition, even when as a solubilizer of tamsulosin, ethanol or glycols are used, these solubilizers are not satisfactory in performance of dissolving tamsulosin, so that the use of a solubilizer having higher solubility has been desired.
Further, even when as an agent for promoting percutaneous absorption, a higher fatty acid ester is used, the agent fails to satisfy performance of percutaneous absorption of tamsulosin.
Accordingly, for developing a transdermal patch of tamsulosin having high skin permeability, it is task to consist of the adhesive layer itself with high performance for dissolving tamsulosin to combine with enhancer with performance for promoting the skin permeability.

The present invention has been completed by taking into consideration the above-described circumstances and the object of the present invention is to provide a tamsulosin-containing transdermal patch capable of being directly applied to the skin, excellent in the percutaneous absorbability of tamsulosin and effective for improving dysuria.

### [Means for Solving the Problem]

The present inventors have found that in the constitution of an adhesive layer of a tamsulosin-containing transdermal patch, by combining tamsulosin and a polyoxyethylene alkyl ether playing a role in increasing the solubility of tamsulosin or by employing a specific type of a base resin itself of the adhesive having high performance of dissolving tamsulosin, the solubility of tamsulosin in the adhesive layer is drastically enhanced.
Further, it has been also found that in the above-described constitution of the adhesive layer, by combining tamsulosin and a propylene glycol fatty acid ester playing a role in promoting percutaneous absorption of tamsulosin, the permeability of tamsulosin into the skin is drastically enhanced.
In addition, it has been also found that by converting at least a part of tamsulosin itself into a free form, the aforementioned improvement of the solubility and skin permeability of tamsulosin and consequently, the effective improvement of the percutaneous absorptivity of tamsulosin can be enhanced.

In other words, the present invention relates to a transdermal patch containing: a backing-layer; and an adhesive layer on the backing-layer, characterized in that the adhesive layer contains an acrylic adhesive, and further contains tamsulosin as an active ingredient in an amount of 0.1 to 20% by mass of the total mass of the adhesive layer, and a polyoxyethylene alkyl ether as an additive.
In the transdermal patch, the polyoxyethylene alkyl ether as an additive is preferably polyoxyethylene lauryl ether.

In addition, the present invention relates to a transdermal patch containing: a backing-layer; and an adhesive layer on the backing-layer, characterized in that the adhesive layer contains an acrylic adhesive based on a copolymer produced by copolymerizing a monomer having a pyrrolidone ring and a (meth)acrylic acid alkyl ester with a carbon atom number of the alkyl group of 4 to 12, and further contains tamsulosin as an active ingredient in an amount of 0.1 to 20% by mass of the total mass of the adhesive layer.

Further, the present invention relates to a transdermal patch containing: a backing-layer; and an adhesive layer on the backing-layer, characterized in that the adhesive layer contains an acrylic adhesive or a synthetic rubber adhesive, and further contains tamsulosin as an active ingredient in an amount of 0.1 to 20% by mass of the total mass of the adhesive layer, and a propylene glycol fatty acid ester as an additive.
Further, in the transdermal patch, it is desirable that the content of the propylene glycol fatty acid ester as an additive is 2 to 40% by mass of the total mass of the adhesive layer, that the propylene glycol fatty acid ester is propylene glycol monolaurate, that the synthetic rubber adhesive containing a styrene-based elastomer and a hydrogenated rosin, or that the acrylic adhesive based on a copolymer produced by copolymerizing a monomer having a pyrrolidone ring with a (meth)acrylic acid alkyl ester having a carbon atom number of the alkyl group of 4 to 12.
Then, the transdermal patch further contains preferably a polyoxyethylene alkyl ether as an additive, more preferably polyoxyethylene lauryl ether as the polyoxyethylene alkyl ether.

Then, in these transdermal patches, it is particularly preferred that the tamsulosin exists in a free form in the adhesive layer or that the tamsulosin exists in a salt form together with an additive having a base in the adhesive layer, and at least a part of the salt is converted into a free form.

### [Effects of the Invention]

Generally, a transdermal patch is easily applied to the skin and is a dosage form suitable for administrating a drug continuously.
According to the present invention, by combining an acrylic adhesive with a polyoxyethylene alkyl ether playing a role in increasing solubility, tamsulosin can be dissolved in a high concentration in the adhesive layer.
In addition, preferably by using polyoxyethylene lauryl ether as the polyoxyethylene alkyl ether, tamsulosin can be dissolved in a higher concentration in the adhesive layer.

In addition, according to the present invention, by using an acrylic adhesive based on an acrylic ester copolymer produced by polymerizing a monomer having a pyrrolidone ring with a (meth)acrylic acid alkyl ester having a carbon atom number of the alkyl group of 4 to 12, tamsulosin can be dissolved in a high concentration in the adhesive layer without using a solubilizer.

Further, according to the present invention, by combining an acrylic adhesive or a rubber adhesive with a propylene glycol fatty acid ester playing a role in promoting percutaneous absorption, the skin permeability of tamsulosin in the adhesive layer can be enhanced.
More preferably, by using propylene glycol monolaurate as the propylene glycol fatty acid ester, by using an adhesive containing a styrene-based elastomer and a hydrogenated rosin as the synthetic rubber adhesive, or by using an adhesive based on a copolymer produced by copolymerizing a monomer having a pyrrolidone ring with a (meth)acrylic acid alkyl ester having a carbon atom number of the alkyl group of 4 to 12 as the acrylic adhesive, tamsulosin can be dissolved in a high concentration in the adhesive layer.
Further, by combining with the polyoxyethylene alkyl ether playing a role in increasing the solubility, particularly with polyoxyethylene lauryl ether as the polyoxyethylene alkyl ether, tamsulosin can be dissolved in a further higher concentration in the adhesive layer.

Further, by causing at least a part of the tamsulosin in the adhesive layer to exist in a free form, tamsulosin can be dissolved in a further higher concentration in the adhesive layer.

Thus, according to the present invention, the transdermal patch containing tamsulosin is formed, and tamsulosin dissolved in the adhesive layer is released from the adhesive layer for a long period to permeate the skin efficiently, so that a continuous administration can become possible.
Above all, by incorporating tamsulosin in the adhesive layer in a high concentration, such a disadvantage of transdermal patches containing tamsulosin as lowered permeability of tamsulosin caused by the horny layer in the surface of the skin which has a barrier function against the tamsulosin absorption, can be solved, so that high skin permeability of tamsulosin can be achieved. In addition, by incorporating tamsulosin in the adhesive layer in a high concentration, the release of tamsulosin from the adhesive layer for a long period becomes possible. Further, by incorporating an additive playing a role in promoting the percutaneous absorption, the skin permeability of tamsulosin can be further enhanced.

### BEST MODES FOR CARRYING OUT THE INVENTION

The transdermal patch according to the present invention is produced by disposing an adhesive layer on the surface of a backing-layer and by laminating a release liner on the adhesive layer as covering usually the whole surface of the adhesive layer. Hereinafter, each constitution element of the present invention and the function thereof will be more specifically described.
Here, in the present invention, "based on the total mass of the adhesive layer" should mean that the total mass of the adhesive layer containing an acrylic adhesive or synthetic rubber adhesive, tamsulosin (active ingredient), an optionally blended additive (enhancing the solubility: for example, polyoxyethylene lauryl ether, promoting the percutaneous absorption: for example, propylene glycol monolaurate) and other components (crosslinker, anti-oxidants, filler etc.) is caused to be the standard, with proviso that the standard total mass of the adhesive layer does not include the mass of an organic solvent used for dilution.

### 1) Adhesive layer

The adhesive layer which is a constituting element of the transdermal patch of the present invention contains as essential components, an active ingredient, an adhesive, an additive (which plays a role in increasing solubility and/or promoting percutaneous absorption). However, when the used acrylic adhesive is an adhesive based on a copolymer produced by copolymerizing a monomer having a pyrrolidone ring and a (meth)acrylic acid alkyl ester having a carbon atom number of the alkyl group of 4 to 12, an additive may not be blended.
In addition if desired, the adhesive layer of the transdermal patch may contain further the following other additives for the general use.

### (1) Active ingredient

The active ingredient contained in the adhesive layer of the transdermal patch in the present invention is tamsulosin.
Here, taking into consideration the effect required for the preparation or the type of the below-described additives for playing a role in increasing the solubility or promoting the percutaneous absorption, it is desirable that the amount of the used tamsulosin is 0.1 to 20% by mass, more preferably 1 to 10% by mass based on the total mass of the adhesive layer.
In addition, tamsulosin exists in a free form or a salt form in the adhesive layer. When tamsulosin exists in a salt form, it is desirable to use tamsulosin after a part or the whole of tamsulosin has been converted into a free form by adding a basic additive.
The salts of tamsulosin include pharmaceutically acceptable salts such as acid addition salts in which for example, an inorganic acid such as hydrochloric acid, sulfuric acid and hydrobromic acid or an organic acid such as acetic acid, oxalic acid, maleic acid, fumaric acid, citric acid and lactic acid, and these salts may be used. Particularly, a hydrochloride salt, that is tamsulosin hydrochloride is clinically useful.
In addition, examples of the basic additives used for converting a part or the whole of the drug into a free form include potassium hydroxide, sodium hydroxide, acetic acid salts, monoethanol amine, diethanol amine, diisopropanol amine, trishydroxymethylaminomethane.

### (2) Adhesive

The adhesive contained in the adhesive layer of the transdermal patch of the present invention is an acrylic adhesive or a synthetic rubber adhesive.

### (2)-1 Acrylic adhesive

The acrylic adhesive is desirably composed of a (meth)acrylic acid ester copolymer containing preferably a (meth)acrylic acid alkyl ester having a carbon atom number of the alkyl group of 4 to 12 as an essential monomer component.
Or the acrylic adhesive used in the present invention is desirably composed of a copolymer produced by copolymerizing a monomer having a pyrrolidone ring with a (meth)acrylic acid alkyl ester having a carbon atom number of the alkyl group of 4 to 12.

Examples of the acrylic acid alkyl ester having a carbon atom number of the alkyl group of 4 to 12 include n-butyl acrylate, n-hexyl acrylate, n-octyl acrylate, 2-ethylhexyl acrylate, isooctyl acrylate, isononyl acrylate, n-decyl acrylate, isodecyl acrylate and methacrylic ester such as n-decyl methacrylate, isodecyl methacrylate and lauryl methacrylate.
These (meth)acrylic acid alkyl ester may be used individually or in combination of two or more types thereof.
Among the (meth)acrylic acid alkyl esters, 2-ethylhexyl acrylate, n-octyl acrylate, isooctyl acrylate, isononyl acrylate and lauryl methacrylate are particularly preferred.

Examples of the monomer having a pyrrolidone ring include N-vinyl-2-pyrrolidone and N-vinyl-2-piperidone.
Though these monomers are generally used individually, if necessary, may be used in combination of two or more types thereof.

The (meth)acrylic acid ester copolymer is a copolymer of the (meth)acrylic acid alkyl ester or a copolymer of the monomer having a pyrrolidone ring and a (meth)acrylic acid alkyl ester, thus such a copolymer can exhibit properties as an acrylic adhesive such as low skin irritation.
When the (meth)acrylic acid ester copolymer includes the (meth)acrylic acid alkyl ester as an essential monomer component, the copolymerization ratio thereof is preferably 60 to 99% by mass, more preferably 70 to 98% by mass. At this time, the copolymer may further include the monomer having a pyrrolidone ring as a monomer component of the copolymer and in this case, the copolymerization ratio of the monomer having a pyrrolidone ring is preferably 1 to 40% by mass, more preferably 2 to 30% by mass.

As other monomer components contained in the acrylic ester copolymer, a vinyl monomer having a functional group is preferred. Specific examples thereof include monomers having a hydroxy group such as 2-hydroxyethyl acrylate, 3-hydroxypropyl acrylate and 4-hydroxybutyl acrylate; monomers having a carboxyl group such as acrylic acid, methacrylic acid, maleic acid, maleic anhydride, itaconic acid and monobutyl maleate; monomers having an amino group such as acrylamide, dimethylacrylamide, diethylacrylamide, methacrylamide and N-methylolacrylamide; and monomers having an epoxy group such as glycidyl acrylate and glycidyl methacrylate.
These vinyl monomers having a functional group may be used individually or in combination of two or more types thereof.
The copolymerization ratio of the other monomer components in the acrylic ester copolymer that is a vinyl monomer having a functional group is preferably 20% by mass or less, more preferably 10% by mass or less.

Examples of the other monomer components which can be incorporated in the copolymer and are other than the above-mentioned other monomer components contained in the acrylic ester copolymer include (meth)acrylic acid alkyl esters such as methyl acrylate, ethyl acrylate, propyl acrylate, isobutyl acrylate, t-butyl acrylate, lauryl acrylate, stearyl acrylate, methyl methacrylate, ethyl methacrylate, n-butyl methacrylate, isobutyl methacrylate, t-butyl methacrylate, n-hexyl methacrylate, isooctyl methacrylate, isononyl methacrylate and stearyl methacrylate; vinyl esters such as vinyl acetate; unsaturated nitriles such as acrylonitrile and methacrylonitrile; and vinyl aromatic compounds such as styrene.
The other monomer components which can be incorporated in the copolymer may be used individually or in combination of two or more types thereof.
The copolymerization ratio of the other monomer components which can be incorporated in the acrylic ester copolymer is preferably 30% by mass or less.

The acrylic ester copolymer can be synthesized generally by a radical polymerization. Though as the polymerization method, there can be mentioned a solution polymerization method, an emulsion polymerization method and a mass polymerization method, in terms of capable of obtaining advantageous adhesive properties, the solution polymerization method is preferred.
The polymerization reaction is effected by adding a radical polymerization initiator in an amount of 0.1 to 1% by mass based on the total mass of monomers, in a nitrogen stream at a temperature of 40 to 90°C for a few hours to a few ten hours with stirring. Examples of the used polymerization initiator include organic peroxides such as benzoyl peroxide and lauroyl peroxide; and an azo-based initiator such as azobisisobutyronitrile.

### (2)-2 Synthetic rubber adhesive

The synthetic rubber adhesive is desirably based on preferably a styrene-based elastomer such as a styrene-isoprene-styrene block copolymer (SIS) and a styrenebutadiene-styrene block copolymer (SBS) and a rubber adhesive containing a hydrogenated rosin as a tackifier.

### (3) Additive: additive playing a role in increasing solubility

As an additive playing a role in increasing the solubility of tamsulosin contained in the acrylic adhesive layer of the transdermal patch in the present invention, a polyoxyethylene alkyl ether is used. However, when the (meth)acrylic adhesive is composed of an acrylic ester copolymer produced by polymerizing a monomer having a pyrrolidone ring, the additive may be an arbitrary blended component such as a polyoxyethylene alkyl ether or other additives playing a role in increasing solubility.
As preferable polyoxyethylene alkyl ethers, there can be mentioned those in which the alkyl group has a carbon atom number of 12 to 18. Specific examples thereof include polyoxyethylene lauryl ether, polyoxyethylene cetyl ether and polyoxyethylene stearyl ether, and particularly preferred is polyoxyethylene lauryl ether.
As other suitable additives playing a role in increasing solubility other than the above-mentioned polyoxyethylene alkyl ethers, there can be mentioned propylene carbonate and crotamiton, and these additives may be used in a mixture with a polyoxyethylene alkyl ether.
The content of these additives are preferably 0.1 to 50% by mass, more preferably 0.5 to 20% by mass based on the total mass of the adhesive layer.

### (4) Additive: additive playing a role in promoting percutaneous absorption

Further, in the transdermal patch of the present invention, by using a propylene glycol fatty acid ester as an additive playing a role in promoting percutaneous absorption, needless to say, skin permeability is enhanced in a preparation using an acrylic adhesive, a transdermal patch using a synthetic rubber adhesive which has been construed to have a problem of poor skin permeability becomes achievable.
As preferable propylene glycol fatty acid ester, there can be mentioned those in which the fatty acid ester portion has a carbon atom number of 12 to 18. Specific examples thereof include propylene glycol monolaurate, propylene glycol monopalmitate, propylene glycol monostearate and propylene glycol monooleate, and among them, particularly preferred is propylene glycol monolaurate.
The content of a propylene glycol fatty acid ester which is an additive playing a role in promoting the percutaneous absorption is preferably 2 to 40% by mass, more preferably 20 to 40% by mass based on the total mass of the adhesive layer. When the content is more than 40% by mass, the cohesiveness of the adhesive becomes extremely poor, which can hardly become a property suitable for the transdermal patch. Moreover, when the content is less than 2% by mass, the effect of promoting the percutaneous absorption is lowered.
Examples of the other suitable additives playing a role in promoting the percutaneous absorption other than the propylene glycol fatty acid esters include various higher fatty acid esters, higher fatty acids and higher alcohols and these additives can be used in a mixture with a propylene glycol fatty acid ester.
The content of these other additives playing a role in promoting the percutaneous absorption is preferably 0.1 to 50% by mass, more preferably 0.5 to 20% by mass based on the total mass of the adhesive layer.

### (5) Other additives

The adhesive layer of the transdermal patch in the present invention may further contain, besides the above-mentioned components, other solubilizers, other pharmaceutically acceptable percutaneous absorption promoting agent used usually for a patch, fillers, anti-oxidants or the like.

When an acrylic adhesive is used in the adhesive layer of the transdermal patch in the present invention, for the purpose of enhancing the cohesion of the acrylic adhesive, various crosslinkers may be further added in the adhesive layer.
Examples of the crosslinker include multifunctional isocyanate compounds, multifunctional epoxy compounds and multivalent metal salts.
When the amount of the crosslinker is too small, sufficient cohesion enhancing effect cannot be obtained. In addition, when the amount is too large, the cohesion is enhanced too high and the active ingredient (drug) cannot move smoothly in the adhesive during the application of a patch, so that the drug effectiveness cannot be efficiently exhibited, to which an attention should be paid.

When a synthetic rubber adhesive is used in the adhesive layer of the transdermal patch in the present invention, in addition to a styrene-based elastomer, other natural or synthetic rubber adhesives can be used in an appropriate combination thereof.
Further, for imparting adhesion, the adhesive layer may contain a tackifier agent other than hydrogenated rosin, a softener or the like.

### 2) Backing-layer

With the transdermal patch of the present invention, the end product can be produced by a method including: obtaining a mixture (adhesive) by blending an active ingredient (tamsulosin), an adhesive (acrylic adhesive or rubber adhesive) and if necessary, additives (polyoxyethylene alkyl ether, propylene glycol fatty acid ester) or the like; applying the obtained mixture onto an appropriate release liner; laminating an appropriate backing-layer thereon; and if necessary, cutting into an appropriate size.
The backing-layer is appropriately selected in accordance with the use purpose by taking into consideration following performance to the affected area, self-supporting property during the application, flexibility, stretchability and thickness.
Examples of the backing-layer include papers such as impregnated papers, coated papers, quality papers, kraft papers, Japanese papers and glassine papers; plastic films such as polyester films, polyethylene films, polypropylene films, polyvinyl chloride films, polycarbonate films, polyurethane films and cellophane films; cloth bases such as non-woven cloths, woven cloths and fabrics containing foams, polyester fibers, polyethylene fibers and polypropylene fibers; and laminates thereof. Among them, preferred are, in terms of the stretchability non-woven cloths, woven cloths and fabrics, in terms of usability plastic films having transparency.
The thickness of the used backing-layer is preferably 10 µm to 1000 µm, more preferably 10 µm to 700 µm when the backing-layer is a non-woven cloth, woven cloth and fabric, and is preferably 5 µm to 200 µm, more preferably 5 µm to 100 µm when the backing-layer is a plastic film.
Here, as the backing-layer, one type of the non-woven cloth, woven cloth, fabric and plastic film is used or preferably a laminate of two types or more thereof is used.

### 3) Release liner

The release liner used in the transdermal patch of the present invention is appropriately selected in accordance with the use purpose by taking into consideration property of easy peeling from the adhesive layer, air permeability, water permeability, flexibility, or the like. As the release liner, preferably used is a film composed of polymer materials such as polyethylene, polypropylene and polyester and for enhancing the peeling property, the film surface can be subjected to a silicone treatment or a fluorocarbon treatment to use.

### 4) Production method of transdermal patch

When the acrylic adhesive used in the adhesive layer of the transdermal patch in the present invention, that is the acrylic ester copolymer is synthesized by the solution polymerization method, the acrylic adhesive becomes obtained after polymerization as a solution containing the acrylic ester copolymer. Since this solution as it is or after diluted with an appropriate organic solvent can be used in the production of the transdermal patch of the present invention as "acrylic adhesive solution", a solution coating method is preferably used.
In the solution coating method, first a solution in which an acrylic ester copolymer (adhesive), tamsulosin (active ingredient) and if necessary additives (polyoxyethylene alkyl ether, propylene glycol fatty acid ester), further if desired a crosslinker, other solubilizers, other pharmaceutically acceptable usual agents for promoting the percutaneous absorption, fillers, anti-oxidants are added, is prepared. Into this solution, an organic solvent is added as a diluent to adjust appropriately the concentration of the solution.
As the organic solvent used here, there can be mentioned n-hexane, toluene, ethyl acetate, acetone, methyl ethyl ketone or the like, and the concentration of the acrylic ester copolymer in the solution diluted by these organic solvents is preferably 10 to 50% by mass, more preferably 20 to 40% by mass.
Next, the solution (diluted solution) containing each component is stirred to dissolve, and disperse homogeneously. The thus obtained solution is coated homogeneously on, for example a release liner (polyethylene film subjected to a silicone treatment) using a coating machine such as a knife coater, a comma coater or a reverse coater.
After the coating, the coated release liner is retained in a dry and hot atmosphere maintained at about 40°C to 130°C for about 30 seconds to 10 minutes to volatilize the organic solvent. Depending on the type of the used organic solvent and the thickness of the coated adhesive, the drying condition is appropriately selected.

When a synthetic rubber adhesive is used in the adhesive layer, the adhesive layer is formed by a method including: dissolving in the organic solvent such as n-hexane, a styrene-based elastomer (adhesive), a hydrogenated rosin (tackifier) and additives (polyoxyethylene alkyl ether, and propylene glycol fatty acid ester), further if desired other tackifier, other pharmaceutically acceptable usual agents for promoting the percutaneous absorption, softeners, fillers, anti-oxidants; adding tamsulosin (active ingredient) into the resultant synthetic rubber adhesive solution and mixing the solution; and coating the resultant solution on a release liner etc. using a solution coating method like the case of the above-mentioned acrylic adhesive solution.
Alternatively, the adhesive layer can be formed also by a method like a hot melt or calendar method including: mixing all of the components (including tamsulosin, excluding organic solvent); mixing and stirring the resultant mixture in a nitrogen stream while heating; and coating the resultant mixture on a release liner etc.

By laminating the backing-layer on the surface of the adhesive layer obtained by the above method, the transdermal patch can be obtained. Depending on the type of the backing-layer, the release liner may be laminated on the surface of the adhesive layer after forming the adhesive layer on the backing-layer.

### Examples

Hereinafter, the present invention is described more specifically referring to Examples, however, which should not be construed as limiting the scope of the present invention.
In Examples, "%" and "parts" mean "% by mass" and "parts by mass", respectively.

### [Example 1]

96% of 2-ethylhexyl acrylate and 4% of acrylic acid were polymerized using 0.5 parts of a polymerization initiator lauroyl peroxide by an ordinary solution polymerization method to obtain an acrylic adhesive (1) solution (solid content: 35%). To 80 parts (as solid content) of this acrylic adhesive (1), 5 parts of tamsulosin-base and further 15 parts of polyoxyethylene lauryl ether (EMULGEN 104P; manufactured by Kao Corporation) as an additive playing a role in increasing the solubility were added.
The resultant mixture was coated on a 75 µm single surface silicone treated PET (polyethylene terephthalate) film (Filmbyna 75E-0010 No. 23; manufactured by Fujimori Kogyou Co., Ltd.) and was dried at 110°C for 3 minutes so that the adhesive layer has a thickness of 25 µm.
Next, on one surface of the adhesive layer, a 25 µm PET film (Lumirror S 10; manufactured by Toray Industries, Inc.) was laminated to obtain a transdermal patch.

### [Example 2]

A transdermal patch was produced in substantially the same manner as in Example 1, except that the amount of the acrylic adhesive (1) was changed to 82 parts (as solid content) and the amount of tamsulosin-base was changed to 3 parts.

### [Examples 3]

In 15 parts of polyoxyethylene lauryl ether, 5 parts of tamsulosin-base was dissolved and the resultant solution was added to 80 parts (as solid content) of the acrylic adhesive (1) used in Example 1 to stir the resultant mixture to become homogeneous.
Next, a transdermal patch was produced in substantially the same manner as in Example 1.

### [Examples 4]

78% of 2-ethylhexyl acrylate and 22% of N-vinyl-2-pyrrolidone were polymerized using 0.5 parts of a polymerization initiator lauroyl peroxide by an ordinary solution polymerization method to obtain an acrylic adhesive (2) solution (solid content: 35.7%). To 95 parts (as solid content) of this acrylic adhesive (2), 5 parts of tamsulosin-base was added (an additive playing a role in increasing the solubility was not used).
Next, a transdermal patch was produced in substantially the same manner as in Example 1.

### [Example 5]

A transdermal patch was produced in substantially the same manner as in Example 4, except that the amount of the acrylic adhesive (2) was changed to 67 parts (as solid content); the amount of tamsulosin-base was changed to 3 parts; and 30 parts of propylene glycol monolaurate (Rikemal PL-100; manufactured by Riken Vitamin Co., Ltd.) as an additive playing a role in promoting the percutaneous absorption was added.

### [Example 6]

77 parts of a rubber adhesive (31.8 parts of styrene-isoprene-styrene (SIS) block copolymer (Quintac 3520; manufactured by Zeon Corporation), 44.5 parts of a hydrogenated rosin ester resin (Pinecrystal KE311; manufactured by Arakawa Chemical Industries, Ltd.), 0.7 parts of dibutylhydroxy toluene (BHT-F; manufactured by Takeda-Kirin Foods Corporation)) instead of an acrylic adhesive, were dissolved in a mixed solution of toluene and n-hexane and to the resultant solution, 3 parts of tamsulosin-base and 20 parts of propylene glycol monolaurate were added.
The resultant mixture was coated on a 75 µm single surface silicone treated PET (polyethylene terephthalate) film (Filmbyna 75E-0010 No. 23; manufactured by Fujimori Kogyou Co., Ltd.) and was dried at 110°C for 3 minutes so that the adhesive layer has a thickness of 25 µm.
Next, on one surface of the adhesive layer, a 25 µm PET film (product name: Lumirror S 10; manufactured by Toray Industries, Inc.) was laminated to obtain a transdermal patch.

### [Example 7]

A transdermal patch was produced in substantially the same manner as in Example 6, except that the amount of the rubber adhesive was changed to 67 parts (27.6 parts of an SIS block copolymer, 38.7 parts of a hydrogenated rosin ester resin, 0.7 parts of dibutylhydroxy toluene) and the amount of propylene glycol monolaurate was 30 parts.

### [Example 8]

A transdermal patch was produced in substantially the same manner as in Example 6, except that the amount of the rubber adhesive was changed to 57 parts (23.5 parts of an SIS block copolymer, 32.9 parts of a hydrogenated rosin ester resin, 0.6 parts of dibutylhydroxy toluene) and the amount of propylene glycol monolaurate was 40 parts.

### [Comparative Example 1]

A transdermal patch was produced in substantially the same manner as in Example 1, except that as the adhesive, an acrylic adhesive (1) used in Example 1 was used; polyoxyethylene lauryl ether was not added in the adhesive layer; and tamsulosin was mixed in a solution of the adhesive layer in a dispersed (not dissolved) state (95 parts (solid content) of an acrylic adhesive (1), 5 parts of tamsulosin-base).

### [Comparative Example 2]

82 parts of a rubber adhesive (35.5 parts of SIS block copolymer (SIS 5002; manufactured by JSR Corporation), 35.5 parts of a hydrogenated rosin ester resin (Pinecrystal KE311; manufactured by Arakawa Chemical Industries, Ltd.), 10.6 parts of liquid paraffin (Hicall M352; manufactured by Kaneda Co., LTD.), 0.4 parts of dibutylhydroxy toluene (BHT-F; manufactured by Takeda-Kirin Foods Corporation)) were dissolved in a mixed solution of toluene and n-hexane and to the resultant solution, 3 parts of tamsulosin-base and 15 parts of polyoxyethylene lauryl ether (EMULGEN 104P; manufactured by Kao Corporation) were added.
Next, a transdermal patch was produced in substantially the same manner as in Example 1.

### [Comparative Example 3]

A transdermal patch was produced in substantially the same manner as in Comparative Example 1, except that instead of an acrylic adhesive (1), a silicone adhesive (Q7-4501; manufactured by Dow Coming Toray Co., Ltd.) was used and the amounts of the adhesive and tamsulosin-base were changed to 99 parts and 1 part, respectively.

### [Evaluation test method]

The transdermal patches in the Examples 1 to 8 and Comparative Examples 1 to 3 were tested according to the following evaluation test method and the solubility of tamsulosin in various adhesives and the skin permeability of tamsulosin were evaluated.

### (1) Test method of the solubility oftamsulosin-base in various adhesives (Examples 1 to 4 and Comparative Examples 1 to 3)

The solubility of tamsulosin-base in various adhesives at 23°C was visually evaluated. When the transdermal patch was transparent, tamsulosin was evaluated to be in a dissolved state, and when the transdermal patch was opaque state, tamsulosin was evaluated to be in an undissolved state.

### (2) Test method of the skin permeability of tamsulosin (Examples 1 to 8 and Comparative Examples 1 and 2)

The obtained transdermal patch was cut off into a size of 1.77 cm² and using a Franz-type diffusion cell and a skin excised from the abdomen of a hairless mouse (7 weeks old, male), the skin permeability test was performed according to the following procedure.
Using as a receiver solution, a 20% polyethylene glycol #400 aqueous solution (32°C), from the start of the test until 48 hours passed, every 4 hours, 1 ml of the receiver solution was taken as a sample and the same amount of a new receiver solution was filled. To the taken receiver solution, ethanol was added and impurities were removed. Thereafter, the drug concentration in the receiver solution was determined by an LC-MS/MS method and the cumulative amount of skin permeated was calculated. With respect to each sample, the test was three times performed and a mean value was obtained.

### [Evaluation test results]

### (1) Solubility of tamsulosin in various adhesives (Examples 1 to 4 and Comparative Examples 1 to 3)

The evaluation results of the solubility of tamsulosin-base in various adhesives are shown in Table 1.
As is apparent from the results shown in Table 1, the acrylic adhesives containing polyoxyethylene lauryl ether used in Examples 1 to 3 could dissolve tamsulosin-base in such a high concentration as 5% and thus have shown advantageous solubility.
In addition, the acrylic adhesive using a pyrrolidone ring-containing compound (Example 4) could dissolve tamsulosin-base in such a high concentration as 5% without using an additive (polyoxyethylene lauryl ether) playing a role in increasing the solubility of tamsulosin and thus has shown advantageous solubility.
On the other hand, the acrylic adhesive not containing polyoxyethylene lauryl ether used in Comparative Example 1 could dissolve tamsulosin-base in a concentration of 3%, but not in such a high concentration as 5%.
In addition, the rubber adhesive used in Comparative Example 2 could dissolve tamsulosin-base in a concentration of only to 3%, though it contained polyoxyethylene lauryl ether.
Further, the silicone adhesive used in Comparative Example 3 could not dissolve tamsulosin-base in a concentration of only 1%.

[Table 1]

**Table 1: Evaluation of solubility of tamsulosin-base in various adhesives**

| Preparation | Adhesive | Additive | Tamsulosin concentration (%) | Evaluation of solubility |
|---|---|---|---|---|
| Example 1 | acrylic^{*1} | added separately with tamsulosin | 5 | ○ |
| Example 2 | acrylic^{*1} | added separately with tamsulosin | 3 | ○ |
| Example 3 | acrylic^{*1} | added with dissolving tamsulosin | 5 | ○ |
| Example 4 | acrylic^{*2} | None | 5 | ○ |
| Comparative Example 1 | acrylic^{*1} | None | 5 | × |
| Comparative Example 2 | rubber | added separately with tamsulosin | 3 | Δ |
| Comparative Example 3 | silicone | None | 1 | × |

| | | | | |
|---|---|---|---|---|
| Evaluation of solubility (○: in dissolved state, Δ: slightly opaque, ×: in undissolved state) *1: acrylic adhesive (1) using acrylic acid alkyl ester as a monomer *2: acrylic adhesive (2) using pyrrolidone ring-containing compound as a monomer | | | | |

### (2) Skin permeability of tamsulosin (Examples 1 to 8 and Comparative Examples 1 and 2)

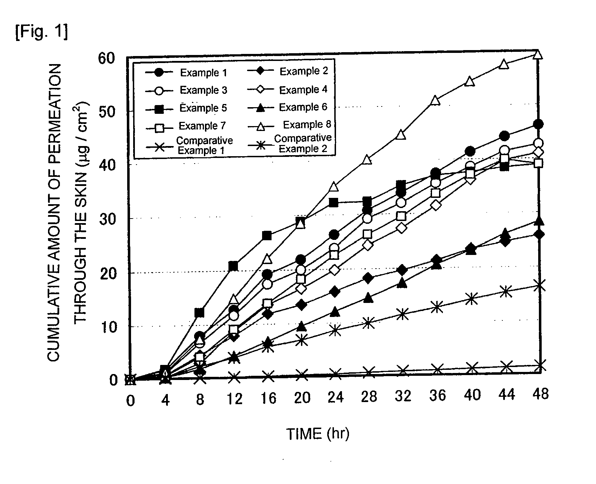

The evaluation results of the skin permeability of tamsulosin-base are shown in Table 2.
As is apparent from the results shown in Table 2 and Fig. 1, it has been shown that the difference in the addition method of tamsulosin in Examples 1 and 3 using an acrylic adhesive (1) has hardly influenced on the skin permeability of tamsulosin. Further, the preparation in which polyoxyethylene lauryl ether which is an additive playing a role in increasing the solubility was added maintained continuously a high skin permeation rate and the cumulative amount of skin permeated has shown a value accepted as sufficient for exhibiting drug effect.
In Example 2, the content of the drug was so small as 3% and the cumulative amount of skin permeated resulted in smaller than those in Examples 1 and 3. However, also in this case, the effect as the transdermal patch resulted in sufficient to be expected. On the other hand, in Comparative Example 1 in which polyoxyethylene lauryl ether was not added, only rather low skin permeability was exhibited in comparison to Examples 1 and 3 in which the concentrations of tamsulosin were the same as that in Comparative Example 1.
The preparation using an acrylic adhesive (2) composed of an acrylic ester copolymer produced by polymerizing a pyrrolidone ring-containing monomer in Example 4 has exhibited relatively advantageous skin permeability even without using an additive playing a role in increasing solubility such as polyoxyethylene lauryl ether.
Further, the preparation using an acrylic adhesive (2) composed of an acrylic ester copolymer produced by polymerizing a pyrrolidone ring-containing monomer in which propylene glycol monolaurate which is an additive playing a role in promoting skin absorption was added in Example 5 has exhibited extremely advantageous skin permeability (though having such a low content of the drug as 3%) and has exhibited the highest skin permeability among in all Examples using an acrylic adhesive.
On the other hand, in Comparative Example 2 using a rubber adhesive, the cumulative amount of skin permeated resulted in lower than that in Example 2 in which the same amount of the drug was used, however, in Examples 6 to 8, by adding propylene glycol monolaurate, the skin permeability was increased and a result which is comparable to that in the case where an acrylic adhesive was used could be obtained. Further, according to the increase of the amount of propylene glycol monolaurate, the skin permeability of tamsulosin was drastically enhanced.

[Table 2]

**Table 2: Result of skin permeation test: cumulative amount of permeation through the skin**

| Time | Cumulative amount of permeation through the skin (µg / cm²) | | | | | | | | | |
|---|---|---|---|---|---|---|---|---|---|---|
| | Example No. | | | | | | | | Comparative Example No. | |
| | 1 | 2 | 3 | 4 | 5 | 6 | 7 | 8 | 1 | 2 |
| 0 | 0 | 0 | 0 | 0 | 0 | 0 | 0 | 0 | 0 | 0 |
| 4 | 1.61 | 0.59 | 0.97 | 0.23 | 1.77 | 0.19 | 0.46 | 1.15 | 0.01 | 0.26 |
| 8 | 7.96 | 4.31 | 6.62 | 2.83 | 12.40 | 1.73 | 4.01 | 7.35 | 0.05 | 2.19 |
| 12 | 12.80 | 7.87 | 11.73 | 8.75 | 20.91 | 4.09 | 9.09 | 14.86 | 0.12 | 3.75 |
| 16 | 19.26 | 11.94 | 17.47 | 13.65 | 26.36 | 6.76 | 13.94 | 22.19 | 0.20 | 5.79 |
| 20 | 21.89 | 13.59 | 20.02 | 16.55 | 28.86 | 9.54 | 18.27 | 28.53 | 0.31 | 6.93 |
| 24 | 26.35 | 15.92 | 23.88 | 19.88 | 32.27 | 12.22 | 22.63 | 35.30 | 0.39 | 8.68 |
| 28 | 30.77 | 18.28 | 29.23 | 24.35 | 32.40 | 14.71 | 26.30 | 40.19 | 0.58 | 9.93 |
| 32 | 33.98 | 19.63 | 32.07 | 27.38 | 35.37 | 17.34 | 29.54 | 44.72 | 0.77 | 11.47 |
| 36 | 37.50 | 21.39 | 35.66 | 31.49 | 37.12 | 20.67 | 33.74 | 51.05 | 0.91 | 12.73 |
| 40 | 41.39 | 23.43 | 38.61 | 36.27 | 37.65 | 23.25 | 37.18 | 54.47 | 1.12 | 14.17 |
| 44 | 44.12 | 24.70 | 41.46 | 39.78 | 38.46 | 26.13 | 39.91 | 57.55 | 1.28 | 15.40 |
| 48 | 46.17 | 25.81 | 42.67 | 41.02 | 38.93 | 28.40 | 42.16 | 59.26 | 1.40 | 16.41 |

### BRIEF DESCRIPTION OF THE DRAWING

[Fig 1] Fig. 1 is a graph showing the results of measuring the cumulative amount of permeation through the skin of the transdermal patch in Examples 1 to 8 and Comparative Examples 1 and 2.

## Claims

1. A transdermal patch comprising:
a backing-layer; and
an adhesive layer on the backing-layer, **characterized in that** the adhesive layer contains an acrylic adhesive, and further contains tamsulosin as an active ingredient in an amount of 0.1 to 20% by mass of the total mass of the adhesive layer, and a polyoxyethylene alkyl ether as an additive.

2. The transdermal patch according to claim 1, wherein the polyoxyethylene alkyl ether as an additive is polyoxyethylene lauryl ether.

3. A transdermal patch comprising:
a backing-layer; and
an adhesive layer on the backing-layer, **characterized in that** the adhesive layer contains an acrylic adhesive composed of a copolymer produced by copolymerizing a monomer having a pyrrolidone ring with a (meth)acrylic acid alkyl ester and a carbon atom number of the alkyl group of 4 to 12, and further contains tamsulosin as an active ingredient in an amount of 0.1 to 20% by mass of the total mass of the adhesive layer.

4. A transdermal patch comprising:
a backing-layer; and
an adhesive layer on the backing-layer, **characterized in that** the adhesive layer contains an acrylic adhesive or a synthetic rubber adhesive, and further contains tamsulosin as an active ingredient in an amount of 0.1 to 20% by mass of the total mass of the adhesive layer, and a propylene glycol fatty acid ester as an additive.

5. The transdermal patch according to claim 4, wherein the content of the propylene glycol fatty acid ester as an additive is 2 to 40% by mass of the total mass of the adhesive layer.

6. The transdermal patch according to claim 4 or 5, wherein the propylene glycol fatty acid ester as an additive is propylene glycol monolaurate.

7. The transdermal patch according to any one of claims 4 to 6, wherein the synthetic rubber adhesive is an adhesive containing a styrene-based elastomer and a hydrogenated rosin.

8. The transdermal patch according to any one of claims 4 to 7, wherein the acrylic adhesive is an adhesive composed of a copolymer produced by copolymerizing a monomer having a pyrrolidone ring and a (meth)acrylic acid alkyl ester having a carbon atom number of the alkyl group of 4 to 12.

9. The transdermal patch according to any one of claims 4 to 8, further comprising a polyoxyethylene alkyl ether as an additive.

10. The transdermal patch according to claim 9, wherein the polyoxyethylene alkyl ether as an additive is polyoxyethylene lauryl ether.

11. The transdermal patch according to any one of claims 1 to 10, wherein the tamsulosin exists in a free form in the adhesive layer or the tamsulosin exists in a salt form together with an additive having a base in the adhesive layer, and at least a part of the salt is converted into a free form.
